# EUROPEAN PATENT SPECIFICATION

(11) **EP 4 494 600 B1**
(45) Date of publication and mention of the grant of the patent: **21.01.2026**
(21) Application number: 23186440.6
(22) Date of filing: 19.07.2023
(51) Int. Cl.: A61F 2/07, A61M 1/14

(54) **VASCULAR ACCESS PROSTHESIS FOR HEMODIALYSIS**
GEFÄSSZUGANGSPROTHESE FÜR DIE HÄMODIALYSE
PROTHÈSE D'ACCÈS VASCULAIRE POUR L'HÉMODIALYSE

(43) Date of publication of application: 22.01.2025
(73) Proprietor: Barone, Hector Daniel, C1240ADP Buenos Aires (AR)
(72) Inventor: Barone, Hector Daniel, C1240ADP Buenos Aires (AR)
(74) Representative: López Camba, Emilia

(56) References cited:
- US-A1- 2004 215 337
- US-A1- 2014 052 231
- US-A1- 2022 088 283

## Description

### BACKGROUND OF THE INVENTION

### FIELD OF INVENTION

The present invention refers to a vascular access prosthesis for cases where the patient carrying the prosthesis needs to be subjected to permanent or temporary treatments involving access to a blood vessel through needles for injection/extraction and circulation of fluids and/or his/her own blood, for example for hemodialysis treatment. Where the prosthesis has a longer useful life unlike conventional vascular accesses thanks to the constructive walls are regenerative and allows the passage of needles without suffering perforations and/or permanent tears, thus providing a greater number of punctures per unit surface for needle insertion into the vascular access.

### DESCRIPTION OF THE PREVIOUS ART

It is widely known that there are health disorders, various diseases, which require treatment to be carried out through access to arterial vessels to inject, extract or circulate a drug, the blood itself of the patient extracted, treated and re-injected, etc. It is often necessary to access an artery in the same body area through perforation, for example the arm, which would generate damage and sometimes local destruction in the arterial wall due to repeated punctures received over time that the prolonged treatment takes. One such case is hemodialysis.

The kidneys act as a waste purification system, removing excess fluids, minerals and waste elements from the blood, being expelled from the body in the form of urine. If a person's kidneys stopped working properly, the waste elements would accumulate in the blood and, in a short time, the person would die.

The most effective treatment for renal failure today is hemodialysis. As it is known in the field of the art, hemodialysis is a treatment for advanced and/or permanent kidney failure that helps to remove wastes, minerals, and excess fluid from the blood when the kidneys cannot do it on their own.

To perform hemodialysis, the patient's blood is removed and passed through a dialysis machine equipped with a special filter called a dialyzer, which cleans or filters the blood that passes through it and then pumps the clean blood and re-enters it into the patient's vascular system. A plurality of flexible hoses or ducts that interconnect the dialysis machine with the patient, allow the blood to be drawn from the patient and returned clean. Patients on permanent dialysis should go to the dialysis center 3 or 4 times a week. This involves perforating the wall of the blood vessel, in the same place or area, twice a session, three times a week which in a short time generates tears and/or injuries in the vascular wall.

In order to avoid damage to the vascular wall, prior to hemodialysis, it is of utmost importance to generate a connection between the patient and the dialysis machine through what is known as "vascular access", in a place in the body from which the blood is drawn and returned, passing through the dialysis machine in between. Vascular access is usually prepared weeks or months before dialysis begins, and there are at least three known types of access: fistula, graft, and catheter.

In relation to the fistula, it is performed with a minor surgery procedure. The fistula is established by attaching a vein to a nearby artery, usually being in the arm. This creates a large blood vessel with a rapid flow of blood. Generally, the preferred place to establish the fistula is the wrist or elbow. The fistula usually takes a few months to establish or enlarge before it can be used and has a useful life of a few years. This allows enough time for the fistula to be ready when treatment is needed.

On the other hand, the graft is another alternative for vascular access. A vascular surgery procedure places an artificial tube between a vein and a nearby artery. The graft is placed on the inside of the elbow or on the arm. Sometimes, grafts can be placed on the leg or chest wall. In general, it is necessary that at least two weeks have passed after surgery to be able to use them.

Finally, within the three types of vascular access mentioned, catheters are most often used for transient vascular access. They are usually used for a short time in people who need to start dialysis before their fistula is ready. The catheter is removed once the fistula has "matured." Not so, sometimes a catheter is used for a long time because it is not possible to establish a fistula or graft in the patient's body. Catheters are soft or semi-rigid plastic tubes and have two lumens, one to draw blood and the other to return clean blood to the body.

Generally, catheters are only placed at the time when dialysis needs to be started. They are arranged in a large vein, for example in the neck, but sometimes placed in the upper chest. However, catheters have greater problems, such as clot formation and infection, compared to fistulas or grafts. With them the blood flow may not be enough for proper dialysis.

The choice of one of the three vascular accesses mentioned above will be in consensus between the patient and the specialist doctor. Once the vascular access has been made, it requires a series of care to avoid infections and/or clots and that, later, it can fulfill its function to carry out hemodialysis. After the necessary time of "maturation" or "healing" of the vascular access, the patient is ready for dialysis.

For hemodialysis treatment, if the patient has a fistula or graft, two needles will be placed in the vascular access at the beginning of each dialysis session. These needles are connected to the flexible ducts mentioned above that allow the extraction and return of blood. If a catheter is present, the flexible ducts can be connected directly to the catheter without the need for needles.

When the needles or flexible ducts are connected to the catheters, dialysis is performed. The blood is drawn and transported by one of the ducts to the dialysis machine. There, the blood passes through the dialyzer or "artificial kidney" where it is purified. Once cleared, the blood returns clean to the patient's body through the other flexible duct.

Thus, in practice vascular access has proven to work properly, however, serious drawbacks still occur. One of them is related to the number of times per week that the person should undergo the treatment. As has been said, patients undergoing hemodialysis should perform it at least three times a week, with the duration of at least four hours. This implies that the patient must perform multiple "punctures" in the body and consequently, in the vascular access.

On the other hand, the diameter of the perforations suffered by the vascular access due to the needles is between 1.47 mm (17G) and 2.11 mm (14G), so repeated punctures throughout the treatment generate failures in it. If vascular access does not function properly, the patient should receive a new vascular access implanted in another sector of the body to replace it. Likewise, a problem in vascular access can generate the formation of clots or infections, even when the care thereof is correct.

On the other hand, if vascular access is a fistula, it needs at least a few months to mature before it can be used. This may mean that the patient must resort to a transient catheter if urgent treatment is required. When using the catheter, the drawbacks and disadvantages listed above will occur.

In the case of using a graft, it requires at least a few weeks for maturation. However, like the catheter, it is more prone to infection and clot formation. In addition, if a clot forms, the graft requires surgery or other treatment to remove it.

However, whether fistula or graft, in both cases the use of needles is required. The use of needles, as stated above, involves multiple perforations in the wall of the device throughout treatment. As is well known, in order to more easily penetrate the vascular walls and the tissues of the patient, the needles have their ends cut at an angle and their edges are generally sharp. This causes the needles to behave, many times, as true "hole punchers" by cutting and removing a small portion of vascular tissue or the wall of any grafted vascular access. However, even when the needles used do not have sharp edges, the ends of them generate perforations with breaks or tears of the walls of the vascular access. Over time, these multiple perforations become permanent and weaken or destroy the vascular access wall making it unusable. On the other hand, these ruptures, tears and perforations make them more prone to infections and/or coagulations, significantly damaging the hemodialysis treatment and consequently, the health and well-being of the patient. In this way, if the fistula or graft presents inconveniences, surgery should be used for correction or removal and replacement.

As can be seen, conventional vascular access requires a series of special care that must necessarily be met to avoid all the problems described above.

On the other hand, the holder of this document has developed a prosthesis disclosed in the US patent 10912638 B2, for the repair of a main blood vessel that has at least one collateral blood vessel that branches from it. This prosthesis is inserted and fixed intraluminally in the main blood vessel of a patient in order to cover a diseased section of the main blood vessel. The prosthesis comprises a tubular trunk prosthesis and one or more interconnecting prosthesis to connect to one or more collateral blood vessels. The trunk prosthesis in turn comprises a liner in the form of a tube having several adjacent and concentric layers that overlap forming interstices that have a first initial diameter and are aligned and radially expanded towards a second internal diameter to a size that allows said interconnection prosthesis to be inserted into said second diameter and expand towards a third external diameter, so as to allow the formation of a passage through the wall of the trunk prosthesis. Then, a passage is formed through the trunk wall of the prosthesis that allows the interconnecting prosthesis to pass through and be fixed without closing the passage to allow free blood flow from the main blood vessel to the collateral blood vessel. In other words, the passage in the trunk prosthesis closes around the wall of the interconnecting prosthesis but not so much as to choke it and prevent the passage of blood to the collateral vessel.

The inventor of this document tried to use the prosthesis of the US patent 10912638 B2 but found the difficulty that although a needle was allowed to pass without breaking the prosthesis fabric, the hole left by the needle did not close completely when the needle was removed, therefore, this prosthesis did not work for use in a vascular access for hemodialysis. US 2004/215337 A1 describes a self-sealing arteriovenous access graft for chronic hemodialysis access, includes outer textile layer concentrically disposed about inner layer of tube, and intermediate self-sealing layer comprising biocompatible polymer.

In view of the current state of the art available for vascular access in hemodialysis treatment, it would be very convenient to have a new vascular access that may have a constructive structure that allows the insertion of needles continuously and frequently, even in a single puncture site, without the wall of the vascular access being damaged, neither temporary nor permanent, avoiding infections and/or the formation of clots, that may have a longer useful life and that, in turn, may facilitate the steps prior to dialysis.

### SUMMARY OF THE INVENTION

It is therefore an object of the present invention to provide a new vascular access prosthesis for hemodialysis treatment having a construction resistant to multiple and frequent punctures without being damaged, with the ability to reconstitute the integrity of the wall and thus having a longer useful life and preventing infections and/or clot formation by ensuring the necessary amount of dialysis that the patient should receive during treatment.

It is still another object of the present invention to provide a vascular access prosthesis for hemodialysis, comprising the prosthesis a flexible support and a liner made of flexible fabric material defined by a plurality of layers, preferably at least two or three concentric mesh layers having wefts open enough to allow the needles to pass through the defined steps between the meshes, without breaks or tears and yet with the ability to close and reconstitute by internal endothelization in order to guarantee the tightness for the blood.

It is also another object of the present invention to provide a vascular access prosthesis that has at least two or more overlapping layers in such a way as to define a liner or mesh or weft of flexible weave that allows the point or sharp end of a device to pass through it in order to open a passage or channel without breaking the weft, fibers or weave through the steps or channels formed in said weft composed of those two or more layers, thus allowing hemodialysis to be carried out countless times without this causing deterioration of the wall of the prosthesis or graft of the vascular access.

It is still another object of the present invention to provide a vascular access prosthesis, wherein the concentric layers that make up the liner overlap together in order to form a wall of the prosthesis that defines labyrinthine interstices that, once the prosthesis is installed, the prosthesis will they are sealed by fibrin of the patient's blood, wherein these labyrinthine interstices formed by the wefts of each layer are capable of being crossed by a needle to form at least one step through the wall of the prosthesis formed by such plurality of concentric layers.

It is also another object of the present invention to provide a vascular access prosthesis for hemodialysis, wherein the prosthesis is implanted in a part of the body of a patient in order to generate a fluid communication of blood flow between at least one artery and a vein, or within a vein, wherein the prosthesis comprises an expandable flexible stent or support to be secured between that artery and vein, or in the vein, and a liner of patterned woven material comprising a plurality of concentric layers with respect to a longitudinal geometric axis, wherein each of the layers has a sufficiently open weave weft, and they overlap together in order to define a wall of the prosthesis that has labyrinthine interstices capable of being crossed by a needle without breaking, tear or cut to carry out the dialysis process, and being that, these labyrinthine interstices are sealed by fibrin from the patient's blood after removing the needle.

### A BRIEF DESCRIPTION OF THE DRAWINGS

The present invention is illustrated by way of example in the following drawings wherein:
Figure 1 shows a perspective view of the vascular access prosthesis for hemodialysis of the present invention;
Figure 2 shows a perspective and partially cut view of the vascular access prosthesis comprising a flexible support and a plurality of layers of weave having very open mesh or weft, in accordance with the present invention;
Figure 3 shows an exploited and partially cut view of the vascular access prosthesis in accordance with the present invention;
Figure 4 shows a partial slice lateral view of the vascular access prosthesis for hemodialysis of the present invention;
Figure 5a shows a part of the arm of a person where the vascular access prosthesis of the present invention is implanted between a vein and a "U"-shaped artery;
Figure 5b shows a part of the arm of a person where the vascular access prosthesis of the present invention is implanted between a vein and an artery in a straight form;
Figure 6 shows a side-to-side view of a portion of the liner of the vascular access prosthesis, where the passage of the needle can be seen through the interstitial labyrinths that are generated due to the wefts of the layers that make it up, without the need to perforate, tear or break its structure;
Figure 7 is a longitudinal and partially sectioned section that shows an alternative embodiment of the vascular access prosthesis made up of three layers of weave formed from a single zigzag invaginated tubular weave,
Figure 8 shows the vascular access prosthesis of the present invention implanted by endovascular route in a vein in a distal position to the suture of the arteriovenous fistula.

### DETAILED DESCRIPTION OF THE INVENTION

Now referring in detail to the figures, the invention consists of a new vascular access prosthesis for hemodialysis treatment, which can be installed inside the arm or other part of the person's body in order to interconnect an artery with a vein or inside the vein distal to a previously performed fistula. It should be understood that the term "distal position" means an implant position of the prosthesis between the suture of the artery-vein anastomosis and the heart of the patient. The prosthesis of the invention includes a prosthesis with a liner that does not tear, leaves no permanent perforations, does not break or cut when a needle passes through it, such as the needles commonly used in hemodialysis, thus prolonging the life of the prosthesis. These perforations, cuts and/or tears are inevitable with the use of conventional prostheses as mentioned in the prior art section.

In fact, the invention proposes the formation of an interstitial labyrinth, window, passage or opening in the wall of the prosthesis to perform dialysis later without this generating drawbacks for it. This labyrinth, passage, opening or window is closed after dialysis and the needle is removed thanks to the effect of fibrin on the material used for the liner. In this way, the prosthesis is not damaged, but rather its walls are regenerated maintaining their original features, thus allowing countless possible steps or paths through the wall to insert the needles and carry out dialysis, avoiding the inconveniences of known vascular accesses.

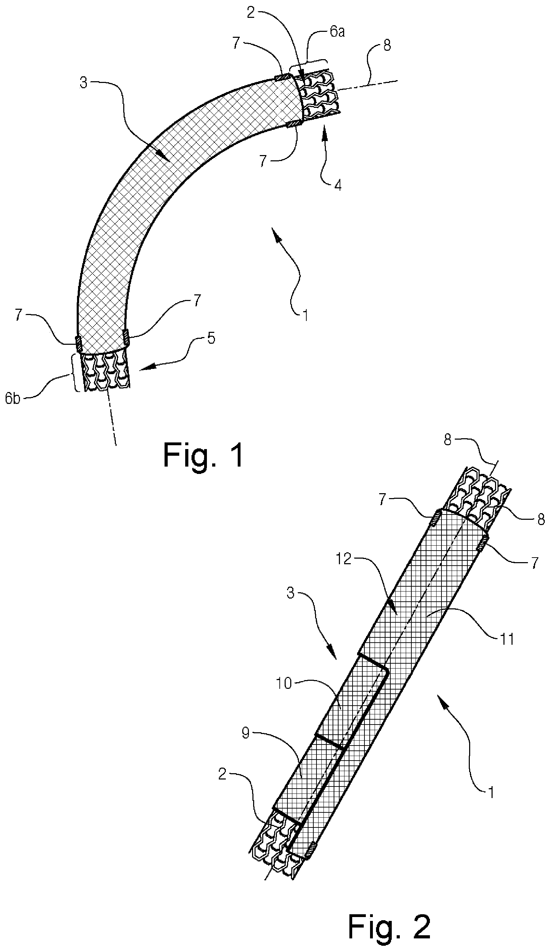

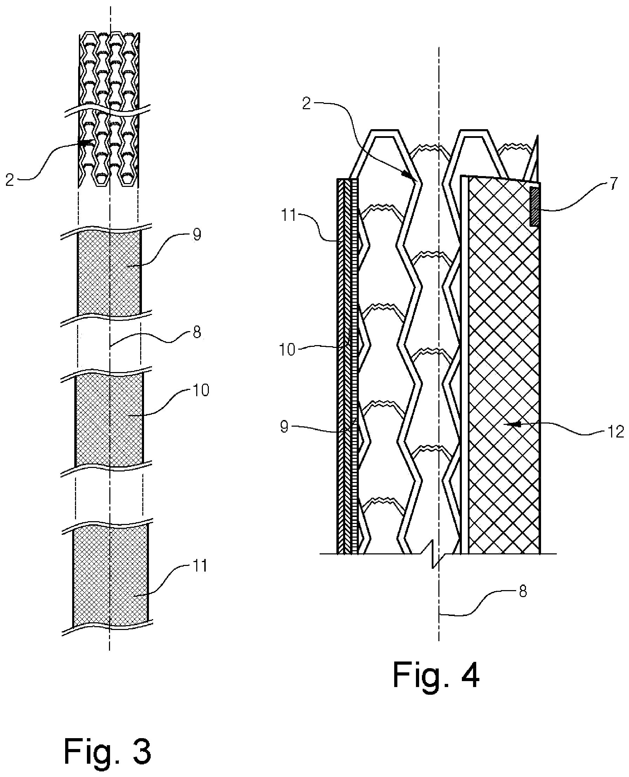

Thus, and according to Figures 1 to 8, a novel vascular access prosthesis for hemodialysis is provided, which is indicated by the general reference 1 and comprises a support 2 and a liner 3 of flexible or compliant fabric made of woven material. Wherein, this support 2 has a cylindrical structure that can include a flexible stent or flexible metal skeleton, of the self-expanding type, which extends along the entire prosthesis 1 accompanying the liner 3.

Flexible stents as means of anchoring are well known in the field of the art of the invention and for such reasons we will not enter into descriptive details about them. The stent of the invention can be made of, but is not limited to, nitinol and has a structure that allows its flexion in any direction so as to generate a "bridge" and anchor between at least one artery and a vein. The flexible stent 2 has two ends, one upper end 4 and one lower end 5, the terms "upper" and "lower" only refer to the position shown in Figure.

The prosthesis of the present invention can be implanted alternately endovascular or surgically. In the case of the endovascular route, the stent 2 has two anchor portions 6a and 6b that protrude beyond the edge of the liner 3 to facilitate the anchoring of the prosthesis within the vein. Likewise, radioopaque markings 7 can be available to facilitate the stent implantation by this route. In the case of the surgical route, the ends of the stent will have prosthesis tubes attached to perform arterial and venous anastomosis respectively as indicated by the standard technique. Therefore, the prosthesis can be sutured to the vascular walls in which case the stent will not need to protrude from the ends of the mesh walls and its function will only be to keep the lumen of the prosthesis open.

For its part, the liner 3 of fabric made of woven material comprises a plurality of layers, at least two, or as illustrated at least three layers, arranged concentrically with respect to a longitudinal geometric axis 8, as best illustrated in Figure 2. According to a preferred embodiment of the invention, such layers comprise an inner layer 9, an intermediate layer 10 and an outer layer 11 which overlap each other defining a wall or membrane 12. It is emphasized that the layers can be independent from each other and overlap so that the structural uniqueness between them is achieved when the prosthesis is expanded with support in the stent 2.

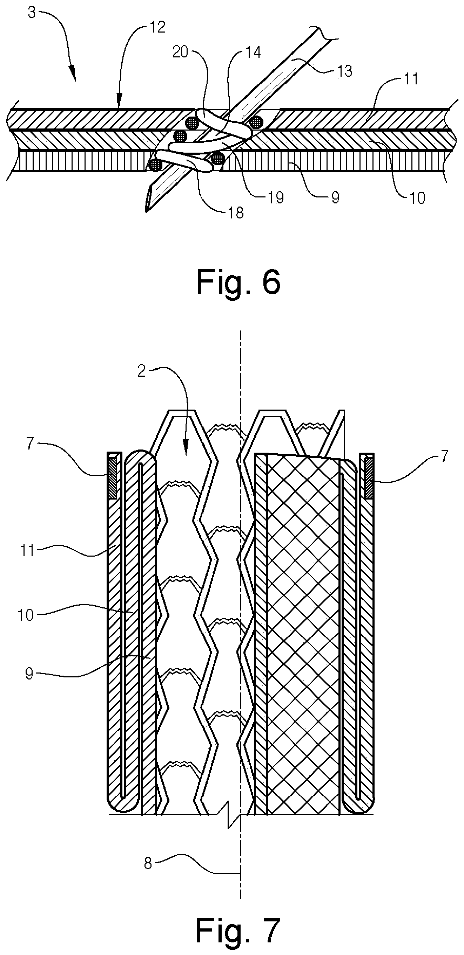

The layers can be separate layers and arranged one inside the other as indicated, or be a single tubular weave arranged in an invaginate, zigzag fashion to define two or more layers for the length of the prosthesis, alternatively three layers such as illustrated in Figure 7. Whether the layers constitute independent weaves or a single weave arranged in zigzag, they may be fixed to the stent or metal skeleton 2 by known techniques and means, such as sutures, clips, etc. Likewise, each of the inner 9, intermediate 10 and outer 11 layers, comprises a weave of any suitable type, for example flat weft and warp weave, knitted weave, etc. The multiple layers that make up the wall or membrane 12 may be of the same type of weave or different weaves but at least one of the layers must contain in their weaves at least one biocompatible elastomeric thread.

According to the concepts of this invention, each layer, separately, presents a weave weft open enough to allow the passage of the needles used in hemodialysis through the passages defined between the meshes, without breaks or tears and, however, with the ability to close, thanks to its elastic construction, and to be reconstituted by internal endothelialization in order to guarantee blood tightness. This is because the present prosthesis defines a three-dimensional elastic matrix or structure that, according to the concepts of the invention, promote the fixation of the fibrin of the blood in the amount necessary to seal its pores or interstices. The sealing of the prosthesis is achieved thanks to the fact that the weave of the layers of the prosthesis is a multifilament polyester weave, which favors the deposit and fixation of the blood fibrin once the prosthesis is installed. Once the wall or membrane 12 is sealed, the prosthesis becomes impervious to blood. The process of fixing the fibrin or sealing is carried out quickly. In the vascular accesses of the previous art, this fixation does not occur because they are made of plastic or polymeric materials already impermeable, and in such materials the fibrin does not find grip support. Unlike this, the layers of the prosthesis of the present invention have elastic elastomeric threads selectively in one or more of them but in order not to impair the settlement and fixation of fibrin.

The porosity of each layer may be the same or different, that is, all layers may have the same weft or different wefts; they may also have equal porosities or different porosities between layers. The layers and conformation of the wall of the prosthesis may be like those of the Argentine patent application P 160103292 of the same holder of this document.

The aforementioned process of fixing the fibrin or sealing can be performed even faster according to a embodiment of the invention wherein one of the layers that make up the wall of the prosthesis consists of a mesh made of bio-compatible elastomeric thread. Thus, if only two layers were used, for example the two layers 9 and 10 of Figures, the elastomeric layer or mesh could be one of the two layers, preferably the outer layer, and in the case that the three layers 9, 10 and 11 are used, the layer or the elastomeric mesh could be one of the two layers, preferably the outer layer. The elastomeric layer would preferably be the intermediate layer 10 of Figures. With the elastomeric layer, the tightness of the prosthesis is recovered faster after a puncture and this is because the threads or strands of the mesh, for example the threads of mesh and warp, open elastically to allow the needle to pass through during puncture and close elastically when the needle is removed, dragging with them the threads of the adjacent layers that are forming an assembly by the deposited fibrin and fixed/grasped to the layers.

As mentioned, at least one of the layers 9, 10 and 11 is a woven mesh with at least one bio-compatible elastomeric thread. It can also be co-woven with an elastomeric thread and one or more non-elastomeric polyester threads.

The non-elastomeric thread, which is polyester, fixes the fibrin while the elastomeric thread only closes the pore opened by the needle. The elastomeric thread leads the rest of the threads to close, that is, it makes them return to their original position of closed weave.

The weaves used in the US patent 10912638 B2, of the same holder, unlike the present invention, lack elastomeric threads and in fact should not have them because the weave of the trunk prosthesis, once opened to form a passage that allows the connection of the interconnecting prosthesis, must be sealed around the interconnecting prosthesis but the threads of the weave must not return to their original position by closing or strangling the interconnecting prosthesis to ensure the free passage of the blood to the collateral artery.

This is opposite to what is sought by the present invention where vascular access presents elastomeric threads that close the open passage through the needle once it is removed.

Another difference with the US patent 10912638 B2 prosthesis is that in this patent the stent can be an expandable or non-expandable balloon, and preferably an expandable balloon, because the stent has to be deformable and the cell, once opened, must remain open so as not to strangle the collateral prosthesis.

In the vascular access of the present invention, the stent 2 is preferably self-expandable, that is, it can move pushed by the needle, but once the needle is withdrawn, it is preferable that it return to its place, thus helping the tissue to close again.

In general, the invention is based on the fact that each of said layers 9, 10 and 11 has a very open weave weft and the layers are concentric so as to overlap together to form a wall or membrane of the prosthesis that, between the wefts of each layer, defines pores or labyrinthine interstices capable of allowing the passage of a needle without breaking the fibers or threads, and after removing the needle the threads return more or less or exactly to their place being the perforation or passage of the needle re-clogged or re-sealed by the endothelial liner already formed, and by the fibrin of the circulating blood that will be deposited later. These pores or interstices between the threads of the weaves of the layers are able to be aligned by means of the entrance of the needle, for example the needles 13 used for dialysis, which when passing through the weft of each layer 9, 10, 11, forms an opening or passage or channel 14 through the wall 12 of the prosthesis 1, as will be explained in relation to Figure 6.

In other words, each layer 9, 10, 11, which forms the wall or membrane 12 of the prosthesis 1 has a physical structure or weave that, once crossed by a tapered, sharp, preferably non-cutting element allows dilation of the pore or interstices between threads or strands that is crossed to a desired or chosen diameter without breaking the fibers. This property is maintained throughout the extension of the membrane 12 once the three layers 9, 10, 11, are radially expanded by the internal metal skeleton or stent 2. The configuration of the cells of the stent 2, once expanded, will allow the passage of the needles. Any of the cells will not limit the passage of the needle through it. If the tip of the needle, when inserted, circumstantially touches one of the stent wires, the wire would only deflect the needle but it would always make its way through the layers meshes. Thus, the 2 stent can be of any type but preferably is a self-expanding stent.

This will allow to cross the wall 12 of the prosthesis 1 and carry out the extraction - return of the blood, without the need to foresee in the prosthesis specific places of puncture, rupture, opening or tearing as happens in the prostheses of the previous art. This novel advantage, of stent and liner, avoids the drawbacks mentioned in the previous art in relation to permanent perforations and detachment of wall material, as well as infections and/or the formation of clots. At the same time, the prosthesis of the present invention can be crossed by the needles as many times as required without this causing damage to future or complications in vascular access, since they cross and move through the labyrinthine interstices formed between the threads or strands or fibers of the layers, avoiding tearing, cutting or perforation of the wall. In this way, once the dialysis is finished and the needles are removed, the wall or membrane of the prosthesis is regenerated by the action of the fibrin of the blood that binds the open pores by the needles. When the specialist has to perform a new dialysis, he/she finds a wall to be punctured regenerated and impermeable with all its pores sealed, having to make new channels or passages through the multilayer mesh with the new punctures, restarting the process described above. Thanks to this property of the prosthesis of this invention, the specialist can puncture at any site of the prosthesis without fear of perforating at a site recently used for fear of causing damage to the structure of the graft wall used as vascular access.

This invention is best illustrated according to the following example, which should not be construed as a limitation imposed on its scope.

### EXAMPLE

A prosthesis was built according to the invention that included:
A nitinol closed cell type stent of 20 cm long and 7 mm diameter developed by the firm Latecba S.A. of Argentina.

The liner was a tubular mesh arranged in the shape of a zigzag forming three concentric layers.

The mesh was attached to the stent by means of braided polyester sutures 4/0, arranged at both ends of the stent and aligned on the longitudinal axis.

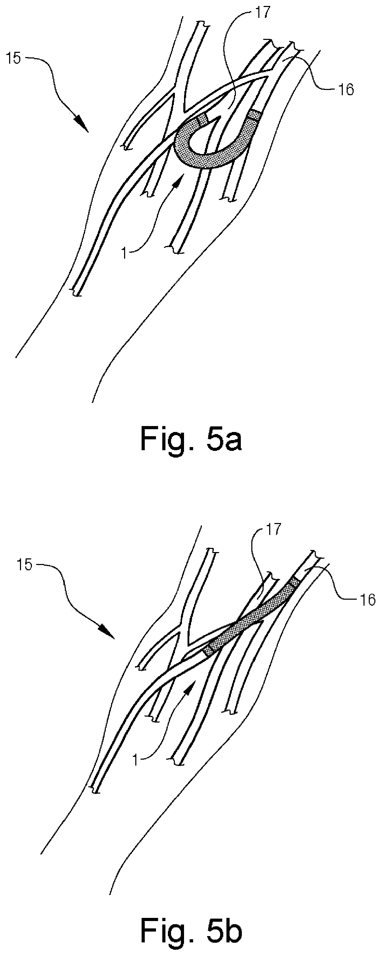

In this way, the prosthesis of the present invention can be implanted in a portion of the inner side of an arm 15 of the patient, and more particularly between an artery 16 and a vein 17, as best illustrated in Figure 5a. Although, in this figure 5a the prosthesis of the invention implanted between an artery and a vein has been illustrated, establishing an interconnection in the form of a "U", this does not imply that the invention is limited to that arrangement, but that, the prosthesis of the invention can be used independently or in conjunction with other vascular access and/or medical devices. For example, Figure 5b illustrates the prosthesis of the invention connecting artery 16 and vein 17 according to a straight interconnection.

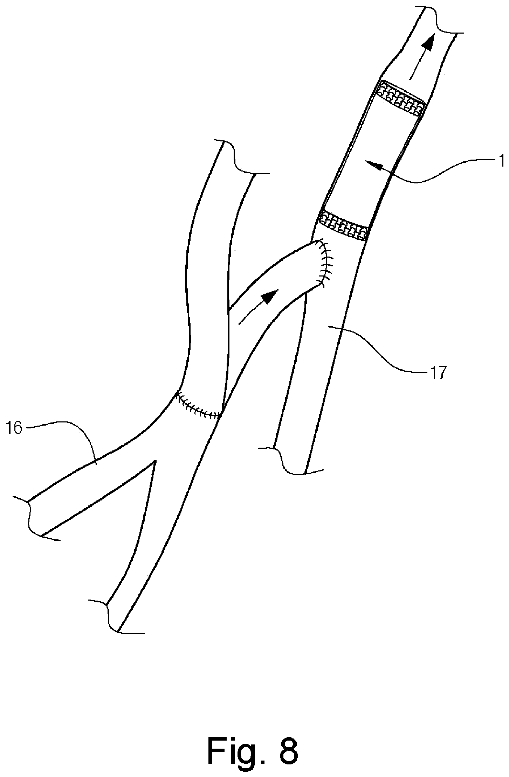

According to another embodiment of the present invention, in Figure 8 a prosthesis 1 of this invention is shown implanted by endovascular route in a vein 17, distal to the arteriovenous anastomosis of the surgical fistula. It should be noted that the aforementioned surgical fistula may be a pre-existing one that was ruled out due to significant damage due to its prolonged use. This invention allows rescuing an arteriovenous fistula through which blood circulates without limitations, but discarded because the constituent material of the prosthesis wall is seriously damaged to withstand new punctures. Taking advantage of the good circulation through the primitive fistula, once installed the prosthesis 1 of the invention, new and future punctures can be made through it.

Once the prosthesis of the invention has been implanted in the arm, or another part of the patient's body, hemodialysis treatment is carried out. The latency time to perform the first dialysis is substantially shorter, only to wait for the three-dimensional structure of membrane and fibrin to form, which will clog the pores avoiding bleeding; being this a much faster process than the maturation of conventional vascular accesses of previous art. This is a significant advantage since the prosthesis of the invention allows dialysis to be performed more quickly after implantation reducing the time of use of the catheter and even avoiding its use completely, thus minimizing many drawbacks as explained.

Continuing with the above, prior to dialysis and as mentioned in the previous art section, the needles 13 pass through the wall or membrane 12 of the prosthesis, particularly the interstices of the wefts of layers 9, 10 and 11 and are arranged within them, displacing the threads or fibers of the wefts of the layers without cutting, breaking, puncturing or tearing such fibers or threads so as to form a passage 14 from side to side of the prosthesis wall 1, as illustrated in Figure 6. As can be seen in this Figure 6, the needle 13 passes between the threads, strands or fibers 18, 19 and 20 of the layers or weaves 9, 10 and 11, without cutting them, that is, it moves them and passes between them. This is possible thanks to the fact that each layer is made of a well-open mesh weave that by itself could not guarantee the impermeability to blood, but combined with the other layers, forms a matrix that closes and is watertight to the blood once the fibrin has been deposited on the wall and the scar tissue with endothelium is formed. This is also possible because one or more of the threads, strands or fibers 18, 19 and 20 is or are elastomeric and/or elastic, and/or composite strands with elastic component. Thus, the end of the needle finds no difficulty in passing between the threads of the first layer, then between those of the second layer and so on through the last. To operate efficiently with the invention it is preferable that the end of the needle 13 does not contain sharp edges that make it operate as "punch". A preferred needle should have a rounded or conical end.

By not cutting, tearing, or piercing the fibers, the structure or weave of each layer is preserved thus remaining in excellent condition to receive future punctures, even in the same place as a previous one. This, as has already been said, diminishes the possibility of future infections and/or clot formation as in the previous art. Then, after dialysis, the needles are removed and the interstices are closed and sealed again thanks to the scar tissue already formed and the incorporation of new fibrin that binds the newly opened pore. According to the variant of the invention described above, this immediate sealing phenomenon could be favored and its time shortened by the incorporation of the layer of elastomeric weave described.

Thus, unlike conventional prostheses and the prior art cited above, the present invention, as clearly described and illustrated, consists of a support covered by a plurality of concentric layers of an expandable weave with a weft sufficiently open to allow the passage of needles without the need for the breakage, punching, cutting or tearing of the meshes of each layer. Once the implant of the prosthesis is finished, the fibrin fixation process begins on and between the different layers of weave, forming an impermeable three-dimensional matrix and allowing blood circulation inside the prosthesis. The fibrin fixation process is much faster than the maturation processes of conventional vascular accesses.

Likewise, when the wall of the prosthesis of the invention regenerates after each perforation, the useful life thereof far exceeds the useful life of the conventional prostheses of the previous art. In turn, hemodialysis treatment is much more practical, and can be carried out as often as necessary without causing damage to vascular access. It is emphasized that the needles only "pierce" the fibrin deposited in the wall of the prosthesis, and move through the labyrinthine interstices of the layers that are arranged as the needles penetrate, without this generating tears, breaks, perforations or cuts in the layers.

Although preferred embodiments of the present invention have been described and illustrated, it will be obvious to those skilled in art that various changes and modifications can be made without departing from the scope of the invention as defined in the attached claims.

## Claims

1. A vascular access prosthesis (1) for hemodialysis, comprising, at least one support or flexible stent (2), and at least one liner (3) made of patterned woven material fixed to said at least one support or stent and comprising a plurality of concentric weave layers (9,10,11) with respect to a longitudinal geometric axis (8), wherein each of the layers has an open weave weft; and the layers are superimposed together in order to define a wall of the prosthesis that presents labyrinthine interstices capable of being crossed by a sharp element without breaking, tearing or cutting, and wherein, these layers of weave together form a fixation matrix for the fibrin of the blood which seals these labyrinthine interstices, wherein at least one of the layers of weave is an elastic layer that includes at least one elastomeric thread.

2. The prosthesis of claim 1, wherein said stent is a self-expanding nitinol stent.

3. The prosthesis of claim 1, wherein said stent extends along the entire at least one liner and protrudes beyond the ends of the at least one liner forming anchoring sections against the wall of a vessel.

4. The prosthesis of claim 1, wherein each layer has the same porosity.

5. The prosthesis of claim 1, wherein the layers have different porosities.

6. The prosthesis of claim 1, wherein all the layers are of the same type of weave.

7. The prosthesis of claim 1, wherein all the layers are of different types of weave.

8. The prosthesis of claim 1, wherein said weave is a knitted weave.

9. The prosthesis of claim 1, wherein said weave is a weft and warp weave.

10. The prosthesis of claim 1, consisting of several layers of weave formed from a single tubular weave invaginated in a zigzag fashion.

11. The prosthesis of claim 1, consisting of several layers of weave independent of each other.

12. The prosthesis of claim 1, wherein said plurality of layers comprises at least two layers, preferably at least three layers.

13. The prosthesis of claim 1, wherein the elastomeric thread is at least one elastic composite thread.

## Patentansprüche

1. Gefäßzugangsprothese (1) für die Hämodialyse, umfassend mindestens einen Träger oder flexiblen Stent (2) und mindestens eine Auskleidung (3), die aus einem gemusterten gewebten Material hergestellt ist, das an dem mindestens einen Träger oder Stent fixiert ist und eine Vielzahl von konzentrischen Gewebelagen (9, 10, 11) in Bezug auf eine geometrische Längsachse (8) umfasst, wobei jede der Lagen einen offenen Gewebeschuss aufweist; und wobei die Lagen zusammen überlagert sind, um eine Wand der Prothese zu definieren, die labyrinthische Zwischenräume aufweist, die von einem scharfen Element durchquert werden können, ohne Bruch, Riss oder Schnitt, und wobei diese Gewebelagen zusammen eine Fixierungsmatrix für das Fibrin des Blutes bilden, das diese labyrinthischen Zwischenräume abdichtet, wobei mindestens eine der Gewebelagen eine elastische Lage ist, die mindestens einen Elastomerfaden beinhaltet.

2. Prothese nach Anspruch 1, wobei der Stent ein selbstexpandierender Nitinolstent ist.

3. Prothese nach Anspruch 1, wobei sich der Stent entlang der gesamten mindestens einen Auskleidung erstreckt und über die Enden der mindestens einen Auskleidung hinaussteht und Verankerungsabschnitte gegen die Wand eines Gefäßes bildet.

4. Prothese nach Anspruch 1, wobei jede Lage die gleiche Porosität aufweist.

5. Prothese nach Anspruch 1, wobei die Lagen unterschiedliche Porositäten aufweisen.

6. Prothese nach Anspruch 1, wobei alle Lagen aus dem gleichen Gewebetyp bestehen.

7. Prothese nach Anspruch 1, wobei alle Lagen aus unterschiedlichen Gewebetypen bestehen.

8. Prothese nach Anspruch 1, wobei das Gewebe ein gestricktes Gewebe ist.

9. Prothese nach Anspruch 1, wobei das Gewebe ein Schuss- und Kettgewebe ist.

10. Prothese nach Anspruch 1, bestehend aus mehreren Gewebelagen, die aus einem einzigen schlauchförmigen, zickzackförmig invaginierten Gewebe gebildet sind.

11. Prothese nach Anspruch 1, bestehend aus mehreren voneinander unabhängigen Gewebelagen.

12. Prothese nach Anspruch 1, wobei die Vielzahl der Lagen mindestens zwei, vorzugsweise mindestens drei Lagen umfasst.

13. Prothese nach Anspruch 1, wobei der Elastomerfaden mindestens ein elastischer Verbundfaden ist.

## Revendications

1. Prothèse d'accès vasculaire (1) pour hémodialyse, comprenant, au moins un support ou une endoprothèse souple (2), et au moins une doublure (3) faite en matériau tissé à motifs fixée audit au moins un support ou endoprothèse et comprenant plusieurs couches de tissage concentriques (9, 10, 11) par rapport à un axe géométrique longitudinal (8), où chacune des couches a une trame à tissage ouvert ; et les couches sont superposées ensemble afin de définir une paroi de la prothèse qui présente des interstices labyrinthiques susceptibles d'être traversés par un élément tranchant sans se briser, se déchirer ou se couper, et dans laquelle, ces couches de tissage forment ensemble une matrice de fixation pour la fibrine du sang qui scelle ces interstices labyrinthiques, dans laquelle au moins l'une des couches de tissage est une couche élastique qui comporte au moins un fil élastomère.

2. Prothèse selon la revendication 1, dans laquelle ladite endoprothèse est une endoprothèse auto-expansive en nitinol.

3. Prothèse selon la revendication 1, dans laquelle ladite endoprothèse s'étend le long de l'ensemble de l'au moins une doublure et dépasse les extrémités de l'au moins une doublure formant des sections d'ancrage contre la paroi d'un vaisseau.

4. Prothèse selon la revendication 1, dans laquelle chaque couche a la même porosité.

5. Prothèse selon la revendication 1, dans laquelle les couches ont différentes porosités.

6. Prothèse selon la revendication 1, dans laquelle toutes les couches sont du même type de tissage.

7. Prothèse selon la revendication 1, dans laquelle toutes les couches sont de différents types de tissage.

8. Prothèse selon la revendication 1, dans laquelle ledit tissage est un tissage tricoté.

9. Prothèse selon la revendication 1, dans laquelle ledit tissage est un tissage de trame et de chaîne.

10. Prothèse selon la revendication 1, constituée de plusieurs couches de tissage formées à partir d'un seul tissage tubulaire invaginé en zigzag.

11. Prothèse selon la revendication 1, constituée de plusieurs couches de tissage indépendantes les unes des autres.

12. Prothèse selon la revendication 1, dans laquelle ladite pluralité de couches comprend au moins deux couches, de préférence au moins trois couches.

13. Prothèse selon la revendication 1, dans laquelle le fil élastomère est au moins un fil composite élastique.
